(19) Europäisches Patentamt
European Patent Office
Office européen des brevets

(11) Veröffentlichungsnummer: **0 594 036 A1**

(12) # EUROPÄISCHE PATENTANMELDUNG

(21) Anmeldenummer: **93116479.2**

(22) Anmeldetag: **12.10.93**

(51) Int. Cl.5: **C07D 417/12**, C07D 413/12, C07D 405/12, C07D 409/12, C07D 311/16, C07D 413/14, A61K 31/37

(30) Priorität: **22.10.92 DE 4235603**

(43) Veröffentlichungstag der Anmeldung:
**27.04.94 Patentblatt 94/17**

(84) Benannte Vertragsstaaten:
**AT BE CH DE FR GB IT LI NL**

(71) Anmelder: **BASF Aktiengesellschaft**
**Carl-Bosch-Strasse 38**
**D-67063 Ludwigshafen(DE)**

(72) Erfinder: **Rendenbach-Mueller, Beatrice**
**Portugieserweg 13**
**D-67435 Neustadt(DE)**
Erfinder: **Karl, Ulrich**
**Gruenerstrasse**
**D-6700 Ludwigshafen(DE)**
Erfinder: **Weifenbach, Harald, Dr.**
**Londoner Ring 71**
**D-6700 Ludwigshafen(DE)**

(54) **Arylalkoxythiocumarine, Verfahren zu ihrer Herstellung und diese enthaltende therapeutische Mittel.**

(57) Arylalkoxythiocumarine der Formel

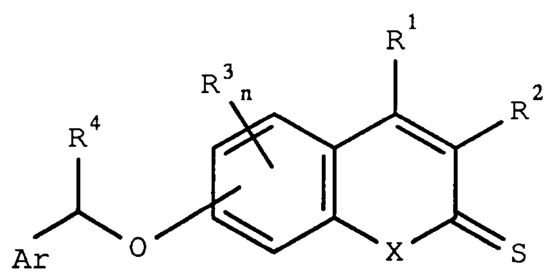

I

in der

| | |
|---|---|
| $R^1$ und $R^2$ | unabhängig voneinander Wasserstoff, $C_1$- bis $C_5$-Alkyl, Trifluormethyl, Phenyl, Halogen oder gemeinsam eine $C_3$-$C_5$-Alkylenkette darstellen; ferner |
| X | Sauerstoff oder Schwefel; |
| $R^3$ | $C_1$- bis $C_5$-Alkyl oder Halogen; |
| n | eine ganze Zahl von 0 bis 3; |
| $R^4$ | Wasserstoff oder $C_1$- bis $C_4$-Alkyl und |
| Ar | Phenyl, das ein- bis zweifach durch Halogen, $C_1$-$C_6$-Alkyl-, $C_3$-$C_7$-Cycloalkyl, $C_1$-$C_6$-Alkoxy-, Nitro, Cyano, Trifluormethyl oder eine Kombination dieser Substituenten substituiert sein kann oder einen heteroaromatischen Ring mit ein bis drei Heteroatomen, die unabhängig voneinander N, O oder S sein können, und der durch $C_1$-$C_6$-Alkyl, $C_3$-$C_7$-Cycloalkyl, $C_1$-$C_6$-Alkoxy, $C_1$-$C_4$-alkoxysubstituiertes $C_1$-$C_6$-Alkyl, 5-6-gliedriges Oxacycloalkyl, Benzyl, $C_1$-$C_5$-Alkoxycarbonyl, Perfluor-$C_1$-$C_2$-alkyl, Phenyl, Halogen substituiert sein kann, Verfahren zu ihrer Herstellung |

EP 0 594 036 A1

und diese enthaltende therapeutische Mittel, insbesondere zur Behandlung zentralnervöser Erkrankungen.

Gegenstand der Erfindung sind neue Phenyl- und Heteroarylalkoxythiocumarine der allgemeinen Formel I, deren Herstellung und therapeutische Mittel, die diese Verbindungen als Wirkstoffe enthalten, insbesondere zur Behandlung zentralnervöser Erkrankungen.

Der Erfindung lag die Aufgabe zugrunde, neue therapeutische Mittel, insbesondere zur Behandlung zentralnervöser Erkrankungen, zu entwickeln.

Die Lösung dieser Aufgabe besteht in den neuen Verbindungen der allgemeinen Formel I nach Anspruch 1, dem Herstellverfahren nach Anspruch 2 und den therapeutischen Mitteln nach den Ansprüchen 3 und 4.

In der allgemeinen Formel I

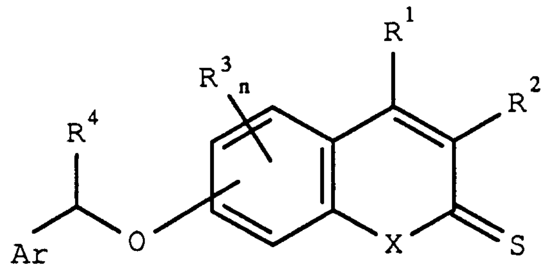
I

bedeuten $R^1$ und $R^2$, die gleich oder verschieden sein können, Wasserstoff, Alkyl von $C_1$ bis $C_5$, wobei $R^1$ und $R^2$ eine gemeinsame Kette von 3-5 Kohlenstoffen bilden können, $CF_3$, Phenyl oder Halogen;
X Sauerstoff oder Schwefel;
$R^3$ Alkyl von $C_1$ bis $C_5$, Halogen;
n eine ganze Zahl von 0 bis 3;
$R^4$ Wasserstoff oder Alkyl von $C_1$ bis $C_4$ und
Ar Phenyl, das ein- bis zweifach durch Halogen, $C_1$-$C_6$-Alkyl, $C_3$-$C_7$-Cycloalkyl, $C_1$-$C_6$-Alkoxy, Nitro, Cyano, Trifluormethyl oder eine Kombination dieser Substituenten substituiert sein kann, oder einen heteroaromatischen, 5- bis 6-gliedrigen Ring mit ein bis drei Heteroatomen, die unabhängig voneinander N, O oder S sein können, und der durch $C_1$-$C_6$-Alkyl, $C_3$-$C_7$-Cycloalkyl, $C_1$-$C_6$-Alkoxy, $C_1$-$C_4$-alkoxysubstituiertes $C_1$-$C_6$-Alkyl, 5- bis 6-gliedriges Oxacycloalkyl, Benzyl, $C_1$-$C_5$-Alkoxycarbonyl, Perfluor-$C_1$-$C_2$-alkyl, Phenyl, Halogen substituiert sein kann.

Mit "Halogen" ist vzgw. Fluor, Chlor oder Brom gemeint.

Die Verbindungen der allgemeinen Formel I lassen sich beispielsweise herstellen, indem man das entsprechende Cumarinderivat der Formel II

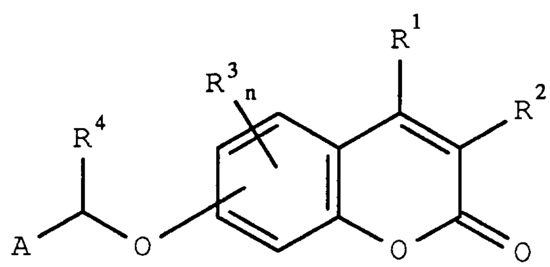
II,

in der $R^1$, $R^2$, $R^3$, $R^4$, n und A die oben angegebenen Bedeutungen haben, mit einem Thiolierungsreagenz in die Schwefelverbindung überführt.

Als Thiolierungsreagenz eignen sich beispielsweise Schwefel, Schwefelwasserstoff und seine Alkalimetall- und Ammoniumsalze, Lawesson's Reagenz (Lawesson, S.O., Org. Synth. 62, 158-164 (1984)), Belleau's Reagenz (Lajoie, G.; Lépine, F.; Maziak L.; Belleau, B., Tetrahedron Lett. 24, 3815 - 3818 (1983)) Phosphorpentasulfid oder aus Phosphorpentasulfid unter Zusatz von Pyridin, Triethylamin, Natrium- oder Kaliumhydrogencarbonat oder Natrium-oder Kaliumcarbonat in situ hergestellte Reagenzien.

Die Umsetzungen können beispielsweise durch Zusammengeben der Cumarine II mit dem Thiolierungsreagenz, vorzugsweise in einem Lösungsmittel, durchgeführt werden.

Als Lösungsmittel sind in Abhängigkeit vom gewählten Thiolierungsreagenz Benzol oder Toluol, Methylenchlorid, Chloroform, Acetonitril, Tetrahydrofuran, Hexamethylphosphorsäuretriamid, Pyridin, Ethanol oder Essigsäure geeignet. Die Reaktionstemperaturen liegen zweckmäßigerweise zwischen der Raumtemperatur und dem Siedepunkt des verwendeten Lösungsmittels.

Die Isolierung und Reinigung der Produkte erfolgt nach an sich bekannten Methoden, beispielsweise durch Umkristallisieren aus einem Lösungsmittel, durch Säulenchromatographie, Extraktion oder gegebenenfalls Überführen in eine Säureadditionsverbindung, sofern A basischen Charakter hat.

Die Verbindungen der allgemeinen Formel II lassen sich beispielsweise herstellen, indem man ein Hydroxycumarin in der Formel III

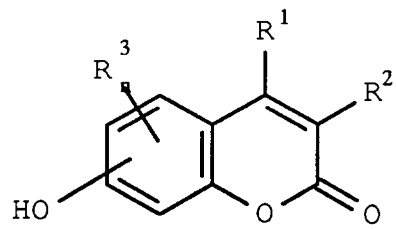

III,

in der $R^1$, $R^2$, $R^3$ und n die oben angegebenen Bedeutungen haben, in an sich bekannter Weise mit einer Verbindung der Formel IV umsetzt,

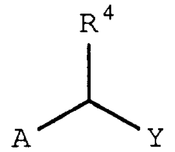

IV,

in der $R^4$ und A wie eingangs definiert sind und Y für eine nucleofuge Abgangsgruppe wie Chlor, Brom oder $R^5SO_2O$ steht. $R^5$ bedeutet hierin Niederalkyl oder gegebenenfalls durch $C_1$-$C_3$-Niederalkyl oder Halogen substituiertes Phenyl. Die Umsetzung kann, wie beispielsweise in Houben-Weyl, Georg Thieme-Verlag, Stuttgart 1965, Bd. 6/3, S. 54 ff. beschrieben, durch Erhitzen der beiden Komponenten, vorzugsweise in Anwesenheit eines inerten Lösungsmittels wie Benzol, Toluol, Methylenchlorid, Aceton, einem niederen Alkohol, Dimethylformamid oder Wasser durchgeführt werden. Die Reaktionstemperaturen liegen zweckmäßigerweise zwischen der Raumtemperatur und dem Siedepunkt des verwendeten Lösungsmittels. Die freiwerdende Säure wird im allgemeinen durch Zusatz von Basen wie Alkali- oder Erdalkalihydroxiden oder -carbonaten oder Aminen wie Pyridin oder Triethylamin abgefangen. Anstelle der Hydroxycumarine der Formel III können auch deren Alkalimetallsalze mit den Verbindungen der Formel IV, vorzugsweise unter wasserfreien Bedingungen in aprotischen Lösungsmitteln wie Ether, Tetrahydrofuran, Dimethylformamid, Dimethoxyethan oder Dimethylsulfoxid, umgesetzt werden. Als Basen können in diesen Fällen Alkalimetallhydride oder -Alkoholate eingesetzt werden. Die Isolierung und Reinigung der Produkte erfolgt nach an sich bekannten Methoden, beispielsweise durch Umkristallisieren aus einem Lösungsmittel, durch Säulenchromatographie oder gegebenenfalls Überführen in eine Säureadditionsverbindung, sofern A basischen Charakter hat.

Die Hydroxycumarine III können nach bekannten Methoden, wie sie zum Beispiel in Elderfield R.C., Heterocyclic Compounds, John Wiley-Verlag, New York 1951, Bd. 2, S. 174 f. beschrieben sind, hergestellt werden, beispielsweise durch Kondensation von Dihydroxybenzolen der Formel V

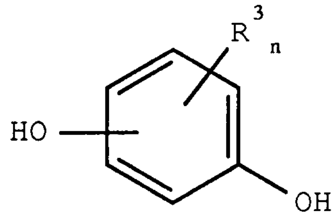

V,

in der $R^3$ und n die oben angegebenen Bedeutungen haben, mit $\beta$-Ketocarbonsäureestern der Formel VI

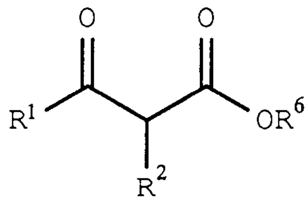

VI,

in der $R^1$ und $R^2$ die oben angegebenen Bedeutungen besitzen und $R^6$ $C_1$-$C_6$-Alkyl bedeutet, in Anwesenheit eines Kondensationmittels wie Schwefelsäure, Phosphorpentoxid oder Aluminiumchlorid.

Die Verbindungen der allgemeinen Formel IV sind entweder bekannt und teilweise kommerziell erhältlich oder lassen sich nach allgemein bekannten chemischen Verfahren herstellen. Methoden zur Synthese von Thiophenderivaten finden sich beispielsweise in: Comprehensive Heterocyclic Chemistry, R. Katritzky und W. Rees, Vol. 4, S. 863 ff., Pergamon Press 1984; Furanderivate z. B. AU579 693, US-S.N. 06/940649 oder Advances in Heterocyclic Chemistry, Vol.30, S. 167 ff., 1982; Thiazolderivate, Oxazol derivate, Isothiazolderivate, Thiadiazolderivate und Oxadiazolderivate z. B. Comprehensive Heterocyclic Chemistry, P. Katritzky und W. Rees, Vol. 6, S. 166, 177, 235, 386, 425 ff., Pergamon Press, 1984; Imidazolderivate z. B. Advances in Heterocyclic Chemistry, Vol. 27, S. 242 ff., 1980; Pyrazolderivate z. B. Heteroaromatic Nitrogen Compounds, The Azoles, S. 31 ff., Cambridge University Press, 1976; Triazolderivate z. B. Comprehensive Heterocyclic Chemistry, R. Katritzky und W. Rees, Vol. 5, S. 733 ff., Pergamon Press, 1984; Isoxazolderivate z. B. GB1560 711 und DE-A-2 754 832.

Falls die Verbindungen der allgemeinen Formel I basisch sind, können sie in die Säureadditionssalze einer physiologisch verträglichen Säure überführt werden. Als übliche physiologisch verträgliche organische oder anorganische Säuren kommen beispielsweise Salzsäure, Bromwasserstoffsäure, Phosphorsäure, Schwefelsäure, Maleinsäure, Fumarsäure, Milchsäure, Weinsäure, Adipinsäure oder Benzoesäure in Betracht. Weitere können aus Fortschritte der Arzneimittelforschung, Bd. 10, S. 224 f., Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem Ether wie Diethyl-oder Methyl-t-butylether, erhalten. Zur besseren Kristallabscheidung können auch Mischungen der genannten Lösungsmittel verwendet werden. Darüber hinaus können gegebenenfalls pharmazeutisch vertretbare wäßrige Lösungen von Säureadditionsverbindungen der erfindungsgemäßen Verbindungen I durch Auflösen der freien Basen in einer wäßrigen Säurelösung hergestellt werden.

Die Verbindungen der allgemeinen Formel I haben Monoaminoxidase (MAO)-inhibierende Aktivität. Aufgrund dessen können sie zur Behandlung von zentralnervösen, insbesondere neurodegenerativen Erkrankungen und Parkinsonismus verwendet werden.

Die MAO hemmende Aktivität der erfindungsgemäßen Verbindungen kann unter Verwendung von Standardmethoden bestimmt werden. So erfolgte die Bestimmung der Monoaminoxidasen A und B in verdünntem Rattenhirnhomogenat, dem 1, unterschiedliche Konzentrationen der zu prüfenden Testsubstanzen und 2. $^{14}C$-Phenylethylamin oder $^{14}C$-Tryptamin in einer Konzentration von 0,4 µmol/l zugesetzt wurden. Dieser Ansatz wurde 20 min bei 37 °C inkubiert.

Die Reaktion wurde dann durch 0,1 normale HCl gestoppt und die Reaktionsprodukte nach Extraktion im Toluol-Szintillator (PPO + POPOP in Toluol) bestimmt. Der Blindwert wurde in analogen Ansätzen mit einer Inkubationszeit von t = 0 min bestimmt.

Aus den bei den verschiedenen Inhibitor-Konzentrationen gegen die Kontrolle ermittelten Hemmwerten wurde durch lineare Regression nach logit-log-Transformation die mittlere Hemmkonzentration (IC50) berechnet.

Die so ermittelte Aktivität einiger erfindungsgemäßer Verbindungen ist aus der folgenden Tabelle ersichtlich:

| Beispiel | $IC_{50}$ [nmol/l] | | $\frac{MAO\ A}{MAO\ B}$ |
|---|---|---|---|
| | MAO A | MAO B | |
| 1 | >10.000 | 1 | >10.000 |
| 2 | >10.000 | 3 | >3333 |
| 3 | >10.000 | 10 | >1000 |
| 4 | ≥10.000 | 2 | >5000 |
| 12 | ≥10.000 | 3 | >3333 |
| 13 | >10.000 | 3 | >3333 |
| 14 | >10.000 | 6 | >1667 |

Die erfindungsgemäßen Verbindungen können in üblicher Weise peroral oder parenteral (subkutan, intravenös, intramuskulär, intraperitoneal) verabfolgt werden.

Die Dosierung hängt vom Alter, Zustand und Gewicht des Patienten sowie von der Applikationsart ab. In der Regel beträgt die tägliche Wirkstoffdosis zwischen etwa 10 und 1000 mg pro Patient und Tag bei peroraler und zwischen etwa 1 und 200 mg pro Patient und Tag bei parenteraler Gabe.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Anitoxidantien und/oder Treibgasen verarbeitet werden (vgl. H. Sukker et al.: Pharmazeutische Technologie, Thieme-Verlag, Stuttgart, 1978). Die so erhaltenen Applikationsformen enthalten den Wirkstoff normalerweise in einer Konzentration von 1 bis 99 Gew.-%.

Beispiele

Allgemeine Herstellvorschrift:

10 mmol Cumarinderivat der allgemeinen Formel II wurden mit 10 mmol (4,04g) Lawesson-Reagenz (2,4-Bis-(4-methoxyphenyl)-1,3-dithio-2,4-diphosphetan-2,4-disulfid) in 20 ml Toluol 1 - 2,5 h bei 90°C gerührt. Nach dem Abkühlen wurde das Lösungsmittel abdestilliert, der Rückstand in DMF aufgenommen und mit Wasser versetzt. Der gebildete Niederschlag wurde abgesaugt und das Rohprodukt durch Umkristallisieren aus Methanol oder durch Säulenchromatographie (Kieselgel, Methylenchlorid) gereinigt.

Nach dieser Vorschrift wurden hergestellt:

Beispiel 1

3,4-Dimethyl-7-(2-isopropyl-1,3,4-thiadiazol-5-yl)-methoxy-2-thiocumarin
Ausbeute: 98 %  Fp.: 134 - 135°C

Beispiel 2

3,4-Dimethyl-7-(2-isopropylthiazol-4-yl)-methoxy-2-thiocumarin
Ausbeute: 66 %  Fp.: 115°C

Beispiel 3

3,4-Dimethyl-7-(2-methyl-1,3,4-oxdiazol-5-yl)-methoxy-2-thiocumarin
Ausbeute: 87 %  Fp.: 187 - 188°C

Beispiel 4

3,4-Dimethyl-7-(2-n-propyl-1,3,4-thiadiazol-5-yl)-methoxy-2-thiocumarin
Ausbeute: 70 % Fp.: 95 - 96 ° C

Beispiel 5

3,4-Dimethyl-7-(1-methyl-5-isopropylpyrazol-3-yl)-methoxy-2-thiocumarin
Ausbeute: 52 % Fp.: 135 - 136 ° C

Beispiel 6

3,4-Dimethyl-7-(2-cyclopropyl-1,3,4-thiadiazol-5-yl)-methoxy-2-thiocumarin
Ausbeute: 76 % Fp.: 145 - 146 ° C

Beispiel 7

7-[3-(1-Methoxyethyl)-isoxazol-5-yl]-methoxy-3,4-dimethyl-2-thiocumarin
Ausbeute: 24 % Fp.: 91 - 92 ° C

Beispiel 8

3,4-Dimethyl-7-(3-n-propylisoxazol-5-yl)-methoxy-2-thiocumarin
Aubeute: 84 % Fp.: 105 - 106 ° C

Beispiel 9

7-[3-(Tetrahydrofuran-3-yl)-isoxazol-5-yl]-methoxy-3,4-dimethyl-2-thiocumarin
Ausbeute: 23 % Fp.: 118 - 119 ° C

Beispiel 10

7-(2-Chlorthien-5-yl)-methoxy-3,4-dimethyl-2-thiocumarin
Ausbeute: 50 % Fp.: 176 - 178 ° C

Beispiel 11

7-Benzyloxy-2-thiocumarin
Ausbeute: 96 % Fp.: 176 - 177 ° C

Beispiel 12

7-(4-Isopropyl)-benzyloxy-2-thiocumarin
Ausbeute: 75 % Fp.: 142 - 143 ° C

Beispiel 13

6-Chlor-3,4-dimethyl-7-(2-isopropyl-1,3,4-thiadiazol-5-yl)-methoxy-2-thiocumarin
Ausbeute: 84 % Fp.: 228 - 229 ° C

Beispiel 14

6-Chlor-3,4-dimethyl-7-(2-cyclopropyl-1,3,4-thiadiazol-5-yl)-methoxy-2-thiocumarin
Ausbeute: 40 % Fp.: 241 - 242 ° C

Beispiel 15

7-(2-Methyl-1,3,4-thiadiazol-5-yl)-methoxy-4-phenyl-2-thiocumarin
Ausbeute: 66 % Fp.: 187 - 188 °C

Beispiel 16

7-(2-Methyl-1,3,4-thiadiazol-5-yl)-methoxy-3,4-tetramethylen-2-thiocumarin
Ausbeute: 41 % Fp.: 159 - 160 °C

Anwendungsbeispiele

A) Auf einer Tablettenpresse werden in üblicher Weise Tabletten folgender Zusammensetzung gepreßt:
40 mg Substanz des Beispiels 1
120 mg Maisstärke
13,50mg Gelatine
45,00mg Milchzucker
2,25mg Aerosil®(chemisch reine Kieselsäure in submikroskopisch feiner Verteilung)
6,75 mg Kartoffelstärke (als 6 %iger Kleister)
B) 20,00 mg Substanz des Beispiels 11
60,00 mg Kernmasse
60,00 mg Verzuckerungsmasse
Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Kalk. Die so hergestellten Dragees werden anschließend mit einem magensaftresistenten Überzug versehen.

**Patentansprüche**

1. Arylalkoxythiocumarine der Formel

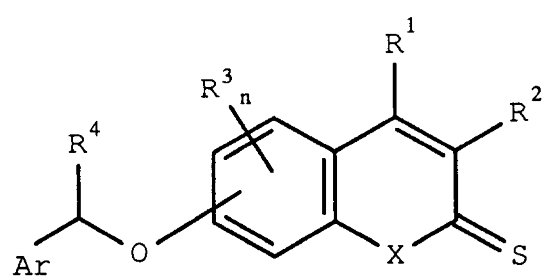

I

in der

- $R^1$ und $R^2$ unabhängig voneinander Wasserstoff, $C_1$- bis $C_5$-Alkyl, Trifluormethyl, Phenyl, Halogen oder gemeinsam eine $C_3$-$C_5$-Alkylenkette darstellen; ferner
- X Sauerstoff oder Schwefel;
- $R^3$ $C_1$- bis $C_5$-Alkyl oder Halogen;
- n eine ganze Zahl von 0 bis 3;
- $R^4$ Wasserstoff oder $C_1$- bis $C_4$-Alkyl und
- Ar Phenyl, das ein- bis zweifach durch Halogen, $C_1$-$C_6$-Alkyl-, $C_3$-$C_7$-Cycloalkyl, $C_1$-$C_6$-Alkoxy-, Nitro, Cyano, Trifluormethyl oder eine Kombination dieser Substituenten substituiert sein kann oder einen heteroaromatischen Ring mit ein bis drei Heteroatomen, die unabhängig voneinander N, O oder S sein können, und der durch $C_1$-$C_6$-Alkyl, $C_3$-$C_7$-Cycloalkyl, $C_1$-$C_6$-Alkoxy, $C_1$-$C_4$-alkoxysubstituiertes $C_1$-$C_6$-Alkyl, 5-6-gliedriges Oxacycloalkyl, Benzyl, $C_1$-$C_5$-Alkoxycarbonyl, Perfluor-$C_1$-$C_2$-alkyl, Phenyl, Halogen substituiert sein kann.

2. Verfahren zur Herstellung der Verbindungen der Formel I nach Anspruch 1, dadurch gekennzeichnet, daß man ein Cumarinderivat der Formel II

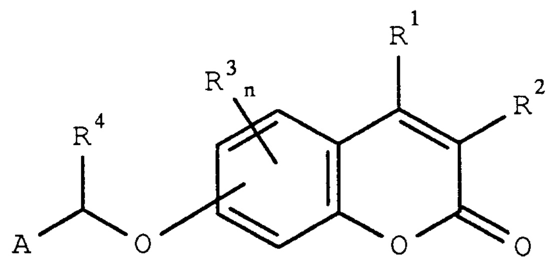

II,

nach bekannten Methoden mit einem Thiolierungsreagenz umsetzt, wobei $R^1$ bis $R^4$, n und A die im Anspruch 1 angegebenen Bedeutungen besitzen.

**3.** Orales therapeutisches Mittel, das als Wirkstoff pro Dosis 10 bis 1000 mg einer Verbindung der Formel I nach Anspruch 1 neben üblichen galenischen Hilfsmitteln enthält.

**4.** Parenterales therapeutisches Mittel, das als Wirkstoff pro Dosis 1 bis 200 mg einer Verbindung der Formel I nach Anspruch 1 neben üblichen galenischen Hilfsmitteln enthält.

**5.** Mittel zur Behandlung von Erkrankungen des zentralen Nervensystems, das als Wirkstoff eine Verbindung der Formel I nach Anspruch 1 neben üblichen galenischen Hilfsstoffen enthält.

Europäisches Patentamt

# EUROPÄISCHER RECHERCHENBERICHT

Nummer der Anmeldung
EP 93 11 6479

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | EP-A-0 363 793 (BASF)<br>* das ganze Dokument *<br>--- | 1-5 | C07D417/12<br>C07D413/12<br>C07D405/12 |
| A | EP-A-0 363 796 (BASF)<br>* das ganze Dokument *<br>----- | 1-5 | C07D409/12<br>C07D311/16<br>C07D413/14<br>A61K31/37 |

RECHERCHIERTE SACHGEBIETE (Int.Cl.5)
C07D

Der vorliegende Recherchenbericht wurde für alle Patentansprüche erstellt

| Recherchenort | Abschlußdatum der Recherche | Prüfer |
|---|---|---|
| DEN HAAG | 18. Februar 1994 | Francois, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.......................................................................................................
& : Mitglied der gleichen Patentfamilie, übereinstimmendes Dokument

EPO FORM 1503 03.82 (P04C03)